(19) Europäisches Patentamt
European Patent Office
Office européen des brevets

(11) Veröffentlichungsnummer: **0 007 319**
**B1**

(12) # EUROPÄISCHE PATENTSCHRIFT

(45) Veröffentlichungstag der Patentschrift: **16.09.81**

(21) Anmeldenummer: **78100479.1**

(22) Anmeldetag: **21.07.78**

(51) Int. Cl.³: **C 07 C 69/95,**
C 07 C 69/76,
C 07 C 121/76,
C 07 C 121/66,
C 07 C 79/46,
C 07 C 101/447

(54) **Neue Indan-1-carbonsäure-Derivate, Verfahren zu ihrer Herstellung, und diese Verbindungen enthaltende pharmazeutische Präparate.**

(43) Veröffentlichungstag der Anmeldung:
**06.02.80 Patentblatt 80/3**

(45) Bekanntmachung des Hinweises auf die Patenterteilung:
**16.09.81 Patentblatt 81/37**

(84) Benannte Vertragsstaaten:
**BE CH DE FR GB LU NL SE**

(56) Entgegenhaltungen:
**DE - A - 2 702 911**
**DE - A - 2 814 556**

(73) Patentinhaber: **SCHERING AKTIENGESELLSCHAFT**
**Berlin und Bergkamen**
**Müllerstrasse 170/178 Postfach 65 0311**
**D-1000 Berlin 65 (DE)**

(72) Erfinder: **Kirsch, Gerald, Dr.**
**Luciusstrasse 6 b**
**D-1000 Berlin 33 (DE)**
Erfinder: **Kapp, Joachim-Friedrich, Dr.**
**Ludolfinger Weg 51**
**D-1000 Berlin 28 (DE)**
Erfinder: **Rufer, Clemens, Dr.**
**Westhofener Weg 14**
**D-1000 Berlin 38 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 007 319 B1

# Neue Indan-1-carbonsäure-Derivate, Verfahren zu ihrer Herstellung und diese Verbindungen enthaltende pharmazeutische Präparate

Die Erfindung betrifft neue Indan-1-carbonsäure-Derivate, ein Verfahren zu ihrer Herstellung und pharmazeutische Präparate, die diese Indan-1-carbonsäure-Derivate als Wirkstoffe enthalten.

Die neuen Indan-1-carbonsäure-Derivate sind gekennzeichnet durch die allgemeine Formel I

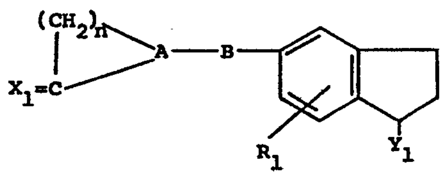

(I),

worin n die Ziffern 2 bis 5, >A—B—

die Gruppierungen >CH—$CH_2$—, >C=CH— oder >CH—CO—,

$R_1$ ein Wasserstoffatom, ein Halogenatom, eine Trifluormethylgruppe, eine Nitrogruppe oder eine Aminogruppe,

$X_1$ zwei Wasserstoffatome oder eine Oxogruppe und

$Y_1$ eine Cyanogruppe, eine Hydroxamidocarbonylgruppe, eine Carbamoylgruppe eine 5-Tetrazolylgruppe, eine Carboxylgruppe, deren Salze mit physiologisch verträglichen Basen, deren Ester von physiologisch unbedenklichen Alkoholen oder deren Amide von physiologisch unbedenklichen Aminen bedeuten.

Unter einem Halogenatom $R_1$ soll vorzugsweise ein Fluoratom, ein Chloratom oder ein Bromatom verstanden werden.

Unter einer niederen Alkylgruppe $R_2$ oder $R_3$ soll vorzugsweise eine 1 bis 4 Kohlenstoffatome enthaltende Gruppe, wie zum Beispiel die Äthylgruppe, die Propylgruppe, die Isopropylgruppe und insbesondere die Methylgruppe verstanden werden.

Die vorliegende Erfindung betrifft gegebenenfalls sowohl die racemischen Phenylessigsäure-Derivate der allgemeinen Formel I, als auch deren optisch aktive Antipoden.

Als physiologisch verträgliche Salze der Carboxylgruppe $Y_1$ seien beispielsweise die Alkali- oder Erdalkalimetallsalze, wie das Natriumsalz oder das Calciumsalz, das Ammoniumsalz, das Kupfer(II)-salz, das Piperazinsalz oder das Methylglukaminsalz, sowie die Salze dieser Verbindungen mit Aminosäuren genannt.

Physiologische unbedenkliche Alkohole, mit denen die Carboxylgruppe $Y_1$ verestert sein kann, sind beispielsweise geradkettige oder verzweigte oder cyclische, gesättigte oder ungesättigte Kohlenwasserstoffreste, die gewünschtenfalls durch ein Sauerstoffatom oder ein Stickstoffatom unterbrochen sein können, oder mit Hydroxygruppen, Aminogruppen oder Carboxylgruppen substituiert sein können, wie zum Beispiel Alkanole, (insbesondere solche mit 1 bis 6 Kohlenstoffatomen) Alkenole, Alkinole, Cycloalkanole, Cycloalkyl-alkanole, Phenylalkanole, Phenylalkenole, Alkandiole, Hydroxycarbonsäuren, Aminoalkenole oder Alkylamino-alkanole und Dialkylaminoalkanole mit 1 bis 4 Kohlenstoffatomen im Alkylrest.

Alkohole, die sich zur Veresterung der Carboxylgruppe eignen, sind beispielsweise solche, die einen Methyl-Carboxymethyl-, Äthyl-, 2-Hydroxyäthyl-, 2-Methoxyäthyl-, 2-Aminoäthyl-, 2-Dimethylaminoäthyl-, 2-Carboxyläthyl-, Propyl-, Allyl-, Cyclopropylmethyl-, Isopropyl-, 3-Hydroxypropyl-, Propinyl-, 3-Aminopropyl-, Butyl-, sek.-Butyl-, tert.-Butyl-, Butyl-2-, Cyclobutyl-, Pentyl-, Isopentyl-, tert.-Pentyl-, 2-Methylbutyl-, Cyclopentyl Hexyl-, Cyclohexyl-, Cyclo-2-enyl-, Cyclopentylmethyl-, Heptyl-, Benzyl-, 2-Phenyläthyl-, Octyl-, Bornyl-, Isobornyl-,Menthyl-, Nonyl-, Decyl-, 3-Phenyl-prop-2-enyl, Undecyl- oder Dodecylrest besitzen. Als zur Veresterung geeignete Alkohole kommen auch solche in Betracht, die zu labilen, d.h. unter physiologischen Bedingungen spaltbaren Estern führen, wie 5-Hydroxyindan, Acyloxymethanole, insbesondere Acetoxymethanol, pivaloyloxymethanol, 5-Indanyloxycarbonylmethanol, Glykolsäure, Dialkylaminoalkanole, insbesondere Dimethylaminopropanol, sowie Hydroxyphtalid.

Als physiologisch unbedenkliche Amine, mit denen die Carboxylgruppe amidiert sein kann, kommen vorzugsweise Alkylamine, Dialkylamine, Alkanolamine, Dialkanolamine mit 1 bis 6 Kohlenstoffatomen im Alkyl- oder Alkanolrest oder fünf- oder sechsgliedrige N-Heterocyclen in Betracht. Als

geeigente Amine seien beispielsweise genannt: das Methylamin, das Äthylamin, das Isopropylamin, das Äthanamin, das Dimethylamin, das Diäthylamin, das Diäthanolamin, das Pyrrolidin, das Piperidin, das Morpholin oder das N-Methylpiperazin.

Das erfindungsgemäße Verfahren zur Herstellung von Indan-1-carbonsäure-Derivaten der allgemeinen Formel Ia

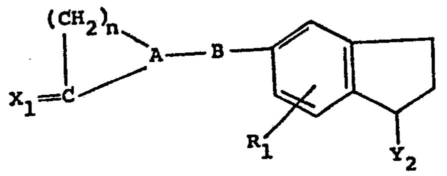

(Ia),

worin n, 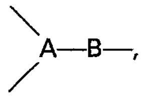,

$X_1$ und $R_1$ die gleiche Bedeutung wie in Anspruch I besitzen und $Y_2$ die gleiche Bedeutung wie $Y_1$ besitzt, aber keine Cyanogruppe oder 5-Tetrazolylgruppe darstellt, ist dadurch gekennzeichnet, daß man in an sich bekannter Weise

a) ein Nitril der allgemeinen Formel II

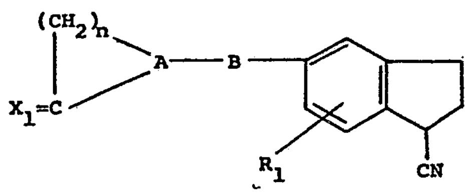

(II),

worin n, 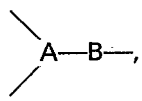,

$X_1$ und $R_1$ die gleiche Bedeutung wie in Anspruch I besitzen hydrolysiert, oder

b) eine Verbindung der allgemeinen Formel III

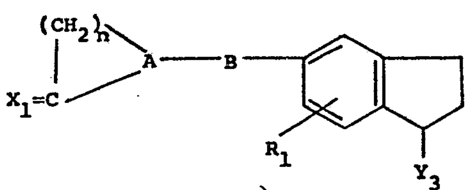

(III),

worin n, 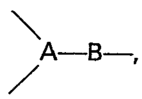,

$X_1$ und $R_1$ die gleiche Bedeutung wie in Anspruch I besitzen und $Y_3$ eine Alkoxycarbonylgruppe, eine Dithianylidengruppe oder eine 4,4-Dimethyl-2-oxazolinylgruppe darstellt, hydrolysiert, oder

c) ein Aldehyd der allgemeinen Formel IV

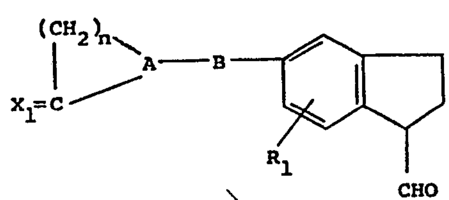

(IV),

worin n, 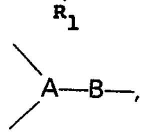,

$X_1$ und $R_1$ die gleiche Bedeutung wie in Anspruch I besitzen oxidiert, oder

d) daß man die Oxogruppe einer Verbindung der allgemeinen Formel V

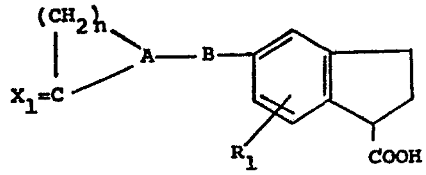

(V),

worin n, 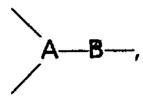

$X_1$ und $R_1$ die gleiche Bedeutung wie in Anspruch I besitzen, wobei mindestens eine der Gruppen A—B— oder $C{=}X_1$ eine Carbonylgruppe bedeutet, durch thermische Behandlung mit Hydrazin reduziert, oder

e) daß man ein Grignard-Reagenz der allgemeinen Formel VI

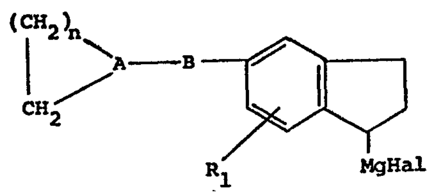

(VI),

worin n, 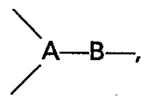

und $R_1$ die gleiche Bedeutung wie in Anspruch 1 besitzen und Hal ein Halogenatom darstellt, mit Kohlendioxid umsetzt, oder

f) daß man eine Verbindung der allgemeinen Formel VII

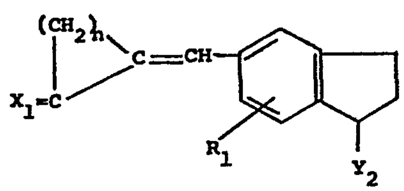

(VII),

worin n, $X_1$, $Y_2$ und $R_1$ die gleiche Bedeutung wie in Anspruch 1 besitzen hydriert, oder

g) daß man eine Verbindung der allgemeinen Formel VIII

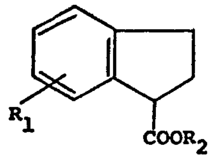

(VIII),

worin

$R_1$ die obengenannte Bedeutung besitzt und $R_2$ ein Wasserstoffatom oder einen Alkylrest mit 1 bis 6 Kohlenstoffatomen darstellt,

in Gegenwart von Friedel-Crafts Katalysatoren mit einem Cycloalkanoylchlorid der allgemeinen Formel IX

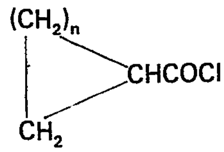 (IX),

worin n die obengenannte Bedeutung besitzt, kondensiert, oder

h) daß man eine Verbindung der allgemeinen Formel X

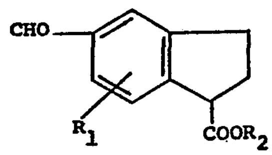 (X),

worin
$R_1$ die obengenannte Bedeutung besitzt und $R_2$ ein Wasserstoffatom der einen Alkylrest mit 1 bis 6 Kohlenstoffatomen darstellt,
mit einem Wittig Reagenz der allgemeinen Formel XI

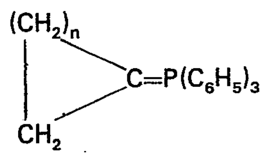 (XI),

worin n die obengenannte Bedeutung besitzt oder mit einer Carbonylverbindung der allgemeinen Formel XII

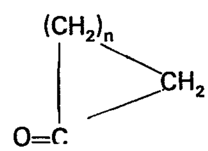 (XII),

worin n die obengenannte Bedeutung besitzt, kondensiert, oder

i) daß man eine Verbindung der allgemeinen Formel XIII

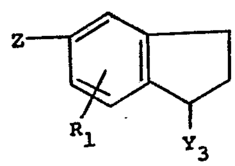 (XIII),

worin
$R_1$ und $Y_3$ die obengenannte Bedeutung besitzen und Z eine Formylgruppe oder eine Cyanogruppe darstellt, mit einer metallorganischen Verbindung der allgemeinen Formel XIV

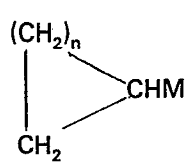 (XIV),

worin n die obengenannte Bedeutung besitzt und M ein Lithiumatom oder eine Magnesiumhalogenidgruppe darstellt umsetzt, oder

j) daß man eine Verbindung der allgemeinen Formel XV

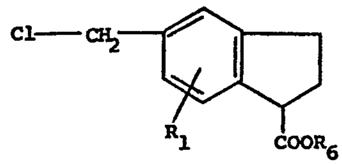

(XV),

worin
$R_1$ und $R_6$ die obengenannte Bedeutung besitzen mit einem $\beta$-Ketoester der allgemeinen Formel XVI

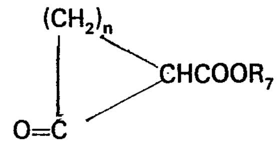

(XVI),

worin n die obengenannte Bedeutung besitzt und $R_7$ eine niedere Alkylgruppe bedeutet, kondensiert, die Estergruppen verseift und die entstandene $\beta$-Ketosäure decarboxyliert
und gegebenenfalls Verbindungen der allgemeinen Formel Ia mit $R_1$ in der Bedeutung eines Wasserstoffatoms halogeniert oder nitriert und die erhaltenen Nitroverbindungen zu Aminoverbindungen reduziert, gegebenenfalls die erhaltenen Carbonsäuren oder reaktionsfähige Derivate derselben in ihre Salze, Ester, Amide oder Hydroxamsäuren überführt.

Das erfindungsgemäße Verfahren zur Herstellung von Indan-1-carbonsäure-Derivaten der allgemeinen Formel Ib

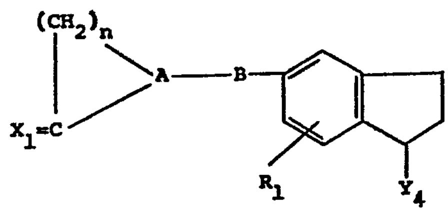

(Ib),

worin n, 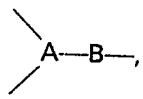

$X_1$ und $R_1$ die gleiche Bedeutung wie in Anspruch 1 besitzen und $Y_4$ eine Cyanogruppe, eine Carbamoylgruppe oder eine 5-Tetrazolylgruppe darstellt ist dadurch gekennzeichnet, daß man in an sich bekannter Weise

k) ein Keton der allgemeinen Formel XVII

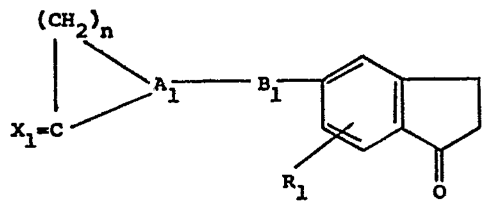

(XVII),

worin n, $X_1$ und $R_1$ die gleiche Bedeutung wie in Anspruch 1 besitzen und 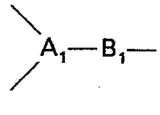

die Gruppierungen 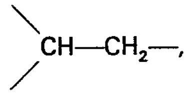 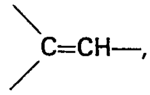 oder 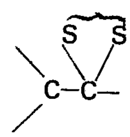

bedeutet mit einem Arylsulfonylmethylisocyanid umsetzt, oder

l) ein Halogenid der allgemeinen Formel XVIII

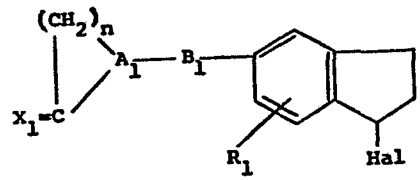

(XVIII),

worin n, 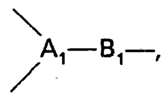

$X_1$ und $R_1$ die obengenannte Bedeutung besitzen und Hal ein Halogenatom darstellt, mit einem Alkalimetall cyanid umsetzt, oder

m) man eine Verbindung der allgemeinen Formel XIX

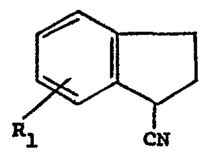

(XIX),

worin
$R_1$ die obengenannte Bedeutung besitzt in Gegenwart von Friedel-Crafts Katalysatoren mit einem Cycloalkanoylchlorid der allgemeinen Formel XX

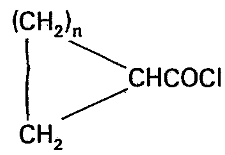

(XX),

worin n die obengenannte Bedeutung besitzt, kondensiert, gegebenenfalls vorhandene thioketalisierte Oxogruppen hydrolysiert und gegebenenfalls die erhaltenen Cyanide der allgemeinen Formel Ib zu den entsprechenden Amiden verseift oder sie in die entsprechenden Tetrazolylverbindunaen überführt.

Das erfindungsgemäße Verfahren gemäß Verfahrensvariante a erfolgt unter den Bedingungen, wie sie dem Fachmann wohl bekannt sind. So kann man die Nitrile beispielsweise mit starken Mineralsäuren (wie Salzsäure oder Schwefelsäure) oder mit starken Basen (wie wässrige Natronlauge oder Kalilauge) partiell zu den entsprechenden Amiden oder unter verschärften Bedingungen zu den entsprechenden Amiden oder unter verschärften Bedingungen zu den entsprechenden Carbonsäuren hydrolysieren.

Für diese Reaktion kann die wasserhaltige Mineralsäure oder Base selbst als Lösungsmittel verwendet werden. Es ist aber andererseits auch möglich, die Reaktion in Gegenwart von polaren Lösungsmitteln wie zum Beispiel niederen Alkoholen (Methanol, Äthanol, Isopropanol etc.), Carbonsäuren (Essigsäure, Propionsäure etc.), polaren Äthern (Glykolmonomethyläther, Dioxan, Tetrahydrofuran etc.). oder dipolaren aprotischen Lösungsmitteln (Dimethylsulfoxyd etc.) durchzuführen.

Üblicherweise wird die Hydrolyse bei einer Reaktionstemperatur von 20°C bis 160°C durchgeführt.

Die für diese Reaktion verwendeten Ausgangsverbindungen der allgemeinen Formel II können, wie bereits erwähnt, gemäß Variante k bis m des erfindungsgemäßen Verfahrens hergestellt werden.

Das erfindungsgemäße Verfahren gemäß Verfahrensvariante b kann ebenfalls in an sich bekannter Weise durchgeführt werden, indem man die Verbindungen der allgemeinen Formel III mittels verdünnter Mineralsäuren (wie zum Beispiel Salzsäure, Schwefelsäure, Phosphorsäure) hydrolysiert. Diese Hydrolyse kann in Abwesenheit zusätzlicher Lösungsmittel durchgeführt werden. Andererseits ist es aber auch beispielsweise möglich, diese Reaktion in Gegenwart polarer Lösungsmittel (so zum Beispiel jener Lösungsmittel, die bei der Beschreibung unpolarer Lösungsmittel wie chlorierter Kohlenwasserstoffe (Dichlormethan, Chloroform, Tetrachloräthan etc.) durchzuführen.

Darüberhinaus können die Ester der allgemeinen Formel III auch mittels basischer Katalysatoren (Kaliumhydrogenkarbonat, Kaliumkarbonat, Kaliumhydroxid, Kaliumäthylat, Natriumkarbonat, Natriumhydroxid, Natriummethylat etc.) hydrolysiert werden, wobei diese Hydrolyse in Gegenwart der gleichen Lösungsmittel durchgeführt werden kann, wie die saure Hydrolyse.

Das erfindungsgemäße Verfahren gemäß Verfahrensvariante b wird üblicherweise bei einer Reaktionstemperatur von $-20$°C bis $+100$°C durchgeführt. Die Herstellung der Ausgangsverbindungen der allgemeinen Formel III mit $Y_3$ in der Bedeutung einer Alkanoyloxygruppe ist in der Beschreibung der Verfahrensvariante h erwähnt.

Das erfindungsgemäße Verfahren gemäß Verfahrensvariante c wird ebenfalls unter Bedingungen durchgeführt, wie sie dem Fachmann wohl bekannt sind. So kann man beispielsweise die Aldehyde der allgemeinen Formel IV in inerten Lösungsmitteln wie zum Beispiel niederen Ketonen (Aceton etc.) oder niederen Carbonsäuren (Essigsäure etc.) oder Wasser mit oxydierenden Schwermetalloxiden (Chrom(VI)-oxid), Natriumdichromat, Kaliumpermanganat, etc.) zu den entsprechenden Carbonsäuren oxydieren. Die für diese Verfahrensvariante benötigten Aldehyde der allgemeinen Formel IV können aus den Ketonen der allgemeinen Formel XX hergestellt werden, indem man diese unter den bekannten Bedingungen (J. Org. Chem., 35, 1970, 1600) mit Chloressigsäureäthylester umsetzt und das gebildete Epoxyd mittels Basen spaltet.

Das erfindungsgemäße Verfahren gemäß Verfahrensvariante d wird unter den Bedingungen durchgeführt, die dem Fachmann unter den Namen Wolff Kishner Reduktion und Huang-Minlon Reduktion bekannt sind.

So kann man beispielsweise die Verbindungen der allgemeinen Formel V in einem hochsiedenden Lösungsmittel (Äthylenglykol, Triäthylenglykol etc.) in Gegenwart von Alkalimetallhydroxyiden (Natriumhydroxid oder Kaliumhydroxid) mit Hydrazin auf 100°C bis 250°C erhitzen und erhält die Verbindungen der allgemeinen Formel Ia.

Die als Ausgangsverbindungen für die Verfahrensvariante d benötigten Verbindungen der allgemeinen Formel V können beispielsweise unter den Bedingungen hergestellt werden, die in den nachfolgenden Ausführungsbeispielen beschrieben sind.

Das erfindungsgemäße Verfahren gemäß Verfahrensvariante e wird unter den für Grignard-Reaktionen bekannten Bedingungen durchgeführt.

So kann man beispielsweise Halogenide der allgemeinen Formel XVIII in einem Ather (Diäthyläther, Diisopropyläther, Di-n-butyläther etc.) mit Magnesium zu dem Grignard-Reagenz der allgemeinen Formel VI umsetzen und auf dieses festes Kohlendioxid einwirken lassen.

Das erfindungsgemäße Verfahren gemäß Verfahrensvariante f wird ebenfalls in an sich bekannter Weise durchgeführt.

So kann man beispielsweise die Verbindungen der allgemeinen Formel VII in einem inerten Lösungsmittel in Gegenwart von Hydrierungskatalysatoren (Raney-Nickel, Platin-oxid-Katalysatoren, Palladiumkatalysatoren etc.) mit Wasserstoff hydrieren. Geeignete inerte Lösungsmittel sind beispielsweise niedere Ester (Essigsäureäthylester etc.), niedere Carbonsäuren (Essigsäure etc.), niedere Alkohole (Methanol, Äthanol, Isopropanol etc.), cyclische Äther (Dioxan, Tetrahydrofuran etc.) oder Wasser.

Die als Ausgangssubstanzen benötigten Verbindungen der allgemeinen Formel VII können beispielsweise gemäß Variante h hergestellt werden, in dem man diese mit p-Toluolsulfonsäurehydrazid umsetzt, das entstandene Hydrat mit Butyllithium behandelt und das entstandene Lithiumsalz mit Kohlenoxid zersetzt (Tetrahedron Letters *34*, 1976, 2947).

Das erfindungsgemäße Verfahrens gemäß Variante g wird unter den üblichen Bedingungen der Friedel-Crafts-Reaktion durch geführt. (Houben-Weyl Band VII/2a, 1973, 38).

So kann man beispielsweise die Verbindungen VIII und IX in einem inerten Lösungsmittel, Schwefelkohlenstoff, Nitromethan oder Nitrobenzol mit einem Friedel-Crafts-Katalysator wie Aluminiumchlorid, Eisen(III)-chlorid, Zinn(IV)-chlorid, Titan(IV)-chlorid Bortrifluorid oder Zinkchlorid umsetzen.

Das erfindungsgemäße Verfahren gemäß Variante h wird unter den Bedingungen durchgeführt, die man üblicherweise bei Wittig-Reaktionen anwendet. ("Organikum"; Organisch chemisches Grundpraktikum-VEB Deutscher Verlag der Wissenschaften, Berlin, 1976, 492). So kann man beispielsweise aus einem Cycloalkyl-triphenyl-phosphoniumhalogenid in einem inerten Lösungsmittel — wie Diäthyläther, Di-isopropyläther, Tetrahydrofuran oder Dimethylsulfoxid — mittels Basen — wie Natriumhydrid oder Butyllithium — das entsprechende Triphenylphosphincycloalkylen herstellen und die so erhaltene Lösung bei −20°C bis 120°C mit dem Aldehyd der allgemeinen Formel X umsetzen.

Die Umsetzung der Aldehyde mit den Carbonylverbindungen der allgemeinen Formel XVI erfolgt unter den für Aldolkondensationen üblichen Bedingungen ("Organikum", 1976, 563), beispielsweise in wässrig-alkoholischer Lösung in Gegenwart von Basen — wie Kaliumhydroxid — oder Säuren — wie Schwefelsäure, Salzsäure oder Essigsäure.

Das erfindungsgemäße Verfahren gemäß Variante i erfolgt unter den üblichen Bedingungen, indem man die metallorganischen Verbindungen der Formel XIV in einem inerten Lösungsmittel — wie Diäthyläther, Diisopropyläther, Dibutyläther oder Tetrahydrofuran aus den entsprechenden Halogeniden durch Umsetzen mit Lithium oder Magnesium herstellt und auf die so erhaltenen Lösungen die Verbindungen der Formel XIII einwirken läßt.

Das erfindungsgemäße Verfahren gemäß Variante j erfolgt ebenfalls unter an sich bekannten Bedingungen, indem man beispielsweise den Ketoester der allgemeinen Formel XVI und die Verbindungen der Formel XV in einem inerten Lösungsmittel, wie Methanol, Äthanol, Dioxan, Glykolmonomethyläther oder Dimethylformamid mit Basen — insbesondere Natrium- oder Kaliumalkoholaten — umsetzt und anschließend mittels Säuren (Schwefelsäure, p-Toluolsulfonsäure etc.) verseift und decarboxyliert.

Die sich gegebenenfalls anschließende Dehalogenierung erfolgt ebenfalls in üblicher Weise, indem man beispielsweise das Halogen hydrierend abspaltet. Dies kann geschehen, indem man die Verbindungen z.B. Äthanol oder Essigsäure in Gegenwart von Platin- oder Palladiumkatalysatoren hydriert.

Die sich gegebenenfalls anschließende Racemattrennung der Säuren erfolgt in üblicher Weise, indem man diese mit optisch aktiven Basen umsetzt und die erhaltenen Diasteromerengemische durch fraktionierte Kristallisation trennt.

Geeignete optisch aktive Basen sind beispielsweise optisch aktive Aminosäuren, d- oder l-1-Phenyläthylamin, d- oder l-Naphtyläthylamin, Brucin, Strychnin oder Chinin.

Die sich gegebenenfalls anschließende Halogenierung von Verbindungen der allgemeinen Formel I a mit $R_1$ in der Bedeutung von Wasserstoff wird in üblicher Weise durchgeführt, indem man auf diese Verbindungen in einem inerten Lösungsmittel (Dichloräthan, Methylenchlorid, Chloroform, Nitrobenzol etc.) in Gegenwart eines Friedel-Crafts-Katalysators (Eisen(III)-chlorid, Eisen(III)-bromid, Aluminiumchlorid etc.) Halogene (Chlor bzw. Brom) einwirken läßt.

Die sich gegebenenfalls anschließende Nitrierung von Verbindungen der Formel I a mit $R_1$ in der Bedeutung von Wasserstoff erfolgt in an sich bekannter Weise, indem man auf diese Verbindungen Salpetersäure beziehungsweise Salpetersäure-Schwefelsäure-Gemische einwirken läßt.

Die sich gegebenenfalls anschließende Reduktion einer vorhandenen Nitrogruppe erfolgt unter den dem Fachmann wohlbekannten Bedingungen (Houben-Weyl Band XI/1, 1957, 360).

Die sich gegebenenfalls anschließende Veresterung der freien Säuren erfolgt ebenfalls nach an sich bekannten Arbeitsmethoden. So kann man die Säuren beispielsweise mit Diazomethan oder Diazoäthan umsetzen und erhält die entsprechenden Methyl- oder Äthylester. Eine allgemein anwendbare Methode ist die Umsetzung der säuren mit den Alkoholen in Gegenwart von Carbonyldiimidazol oder Dicyclohexylcarbodiimid.

Ferner ist es beispielsweise möglich, die Säuren in Gegenwart von Kupfer(I)-oxid oder Silberoxid mit Alkylhalogeniden umzusetzen.

Eine weitere Methode besteht darin, daß man die freien Säuren mit den entsprechenden Dimethylformidalkylacetalen in die entsprechenden Säurealkylester überführt. Weiterhin kann man die Säuren in Gegenwart stark saurer Katalysatoren wie Chlorwasserstoff, Schwefelsäure, Perchlorsäure, Trifluormethylsulfonsäure oder p-Toluolsulfonsäure mit den Alkoholen oder den niederen Alkancarbonsäureestern der Alkohole umsetzen.

Es ist aber auch möglich, die Carbonsäuren in die Säurechloride oder gemischte Säureanhydride zu überführen und diese in Gegenwart basischer Katalysatoren wie Pyridin, Collidin, Lutidin oder 4-Dimethylaminopyridin mit den Alkoholen umsetzen.

Die Salze der Carbonsäuren entstehen beispeilsweise bei der Verseifung der Ester mittels basischer Katalysatoren oder bei der Neutralisation der Säuren mittels Alkalicarbonaten oder Alkalihydroxiden wie zum Beispiel Natriumcarbonat, Natriumhydrogencarbonat, Natriumhydroxid, Kaliumcarbonat, Kaliumhydrogencarbonat oder Kaliumhydroxid.

Ferner ist es möglich, Ester der allgemeinen Formel I in Gegenwart saurer oder basischer Katalysatoren mit dem letztlich gewünschten Alkohol umzusetzen. Hierbei verwendet man als saure oder basische Katalysatoren vorzugsweise Chlorwasserstoff, Schwefelsäure, Phosphorsäure, p-Toluolsulfonsäure, Trifluoressigsäure, beispielsweise Alkali-, Erdalkali- oder Aluminiumalkoholate.

Die sich gegebenenfalls anschließende Amidbildung oder Hydroxamsäurebildung aus den freien Carbonsäuren oder deren reaktionsfähigen Derivaten erfolgt ebenfalls nach den dafür bekannten Verfahren. So kann man beispielsweise die Carbonsäuren unter den bekannten Bedingungen mit Aminen oder Hydroxylamin in Gegenwart von Dicyclohexylcarbodiimid umsetzen, und man erhält die entsprechenden Aminocarbonylverbindungen.

Ferner ist es beispielsweise möglich, die den Carbonsäuren entsprechenden Säurechloride, gemischte Anhydride oder Ester unter den bekannten Bedingungen durch Behandeln mit Ammoniak, mit Aminen oder mit Hydroxylamin in die entsprechenden Amide oder Hydroxamsäuren zu überführen.

Das erfindungsgemäße Verfahren gemäß Verfahrensvariante k kann unter den Bedingungen durchgeführt werden, die dem Fachmann unter dem Namen TOSMIO — Reaktion (Tetrahedron Letters 1973, 1357) bekannt sind.

So kann man die Ketone der allgemeinen Formel XVII beispielsweise in einem polaren Äther (Glykoldimethyläther, Dioxan, Tetrahydrofuran etc.) oder einem dipolaren aprotischen Lösungsmittel (Dimethylformamid, Dimethylsulfoxyd, N-Methylmorpholin, Hexamethylphosphorsäuretriamid etc.) in Gegenwart eines Alkalimetallalkoholates (Natriummethylat, Kaliumäthylat, Kaliumtertiärbutylat etc.) mit Arylsulfonylmethylisocyaniden (insbesondere p-Toluolsulfonylmethylisocyanid) umsetzen und erhält die Verbindungen der Formel Ib.

Die für das erfindungsgemäße Verfahren gemäß Variante k benötigten Ketone der allgemeinen Formel XVII können beispielsweise so hergestellt werden, daß man ein Cycloalkanoylchlorid der Formel IX in Gegenwart von Friedel-Crafts-Katalysatoren unter den Bedingungen der Verfahrensvariante g mit einem entsprechenden Indanon kondensiert.

Das erfindungsgemäße Verfahren gemäß Variante e kann unter den Bedingungen durchgeführt werden, welche man üblicherweise zum Austausch von Halogenatomen gegen eine Cyanogruppe anwendet.

Für diese Verfahrensvariante verwendet man als Ausgangsverbindungen der allgemeinen Formel XVIII vorzugsweise solche Verbindungen, die als Substituenten ein Chlor-, Brom- oder Jodatom tragen.

Diese Reaktion wird vorzugsweise in einem dipolaren, aprotischen Lösungsmittel (wie Dimethylformamid, N-Methylacetamid, N-Methylpyrrolidon, Acetonitril, Dimethylsulfoxyd oder Hexamethylphosphorsäuretriamid) durchgeführt. Als Alkalimetallcyanide verwendet man für diese Reaktion vorzugsweise Natriumcyanid oder Kaliumcyanid.

Bei dieser Umsetzung kann man die Reaktionsgeschwindigkeit signifikant beschleunigen, wenn man die Umsetzung in Gegenwart einen Kronen-Äthers durchführt.

Die Ausgangsverbindungen der allgemeinen Formel XVIII können in üblicher Weise aus den Ketonen der allgemeinen Formel XVII hergestellt werden, indem man diese beispielsweise mit Natriumborhydrid reduziert und die erhaltenen Carbinole mit Halogenwasserstoff, Thionylchlorid, Phosphoroxychlorid, Phosphorpentachlorid etc. umsetzt.

Das erfindungsgemäße Verfahren gemäß Verfahrensvariante m wird unter den gleichen Bedingungen durchgeführt, wie das Verfahren gemäß Variante g.

Die sich gegebenenfalls anschließende Verseifung der Cyanide zu den entsprechenden Amiden wurde bereits in der Beschreibung der Verfahrensvariante a erwähnt.

Zur Darstellung der Tetrazolylverbindungen kann man sich ebenfalls der bekannten Arbeitsmethoden bedienen. So kann man beispielsweise die Nitrile in polaren aprotischen Lösungsmitteln wie Dimethylformamid, N-Methylacetamid, N-Methylpyrrolidon oder Hexamethylphosphorsäuretriamid unter den bekannten Bedingungen mit Alkaliaziden, wie Natriumazid, zu den entsprechenden Tetrazolylverbindungen umsetzen.

Die neuen Indan-1-carbonsäure-Derivate der allgemeinen Formel I sind, wie bereits erwähnt, pharmakologisch wirksame Substanzen, oder Zwischenprodukte zu ihrer Herstellung. Die pharmakologisch wirksamen Verbindungen zeichnen sich insbesondere dadurch aus, daß sie bei systemischer Anwendung eine ausgeprägte antiinflammatorische Wirksamkeit besitzen, eine gute Magenverträglichkeit zeigen, und nur eine relative geringe Toxizität aufweisen. Darüberhinaus zeichnen sich diese Verbindungen oft durch einen raschen Wirkungsbeginn, eine hohe Wirkungsintensität und eine lange Wirkungsdauer aus, sie haben eine günstige Resorbierbarkeit.

Die antiphlogistische Wirksamkeit der erfindungsgemäßen Substanzen kann mit Hilfe des bekannten Adjuvans-Arthritis-Testes ermittelt werden, der wie folgt durchgeführt wird:

Es werden weibliche und männliche Ratten des Stammes Lewis (LEW) in den Gewichtsspanne zwischen 110—190 g verwendet. Die Tiere erhalten Trinkwasser und Altromin-Preßfutter ad libitum.

Für jede Dosisgruppe werden 10 Ratten eingesetzt.

Mycobacterium butyricum der Firma Difco, Detroit, wird als Reizmittel verwandt. Eine Suspension von 0,5 mg Mycobacterium butyricum in 0,1 ml dünnflüssigem Paraffin (DAB 7) wird in die rechte Hinterpfote subplantar injiziert.

Die Testsubstanzen werden vom 11. Versuchstag an täglich über 4 Tage oral gegeben. Die Substanzen werden als klare wässrige Lösung oder als Kristallsuspension unter Zusatz von Myrj 53 (85 mg%) in isotonischer Natriumchlorid-Lösung verabreicht.

Versuchsansatz:

Die Ratten werden in bezug auf ihr Körpergewicht möglichst gleichmäßig in verschiedene Gruppen eingeteilt. Nach plethysmographischer Volumenmessung der rechten Hinterpfote wird in diese subplantar 0,1 ml Adjuvans injiziert. Die rechten Hinterpfoten werden vom 14. Versuchstag bis zum Versuchsende gemessen. Die Versuchsdauer beträgt 3 Wochen.

Bestimmt wird die Dosis an Testsubstanz, bei der eine 40%ige Abheilung beobachtet wird ($=ED_{40}$).

Eine häufige Komplikation bei der Therapie mit nichtsteroidalen Entzündungshemmern stellt das Auftreten von Magenulcerationen dar. Diese Nebenwirkung kann im Tierversuch nachgewiesen werden, wobei als Dosis die Menge Testsubstanz verwendet wird, bei der im Adjuvans-Arthritis Test eine 40%ige Abheilung beobachtet wird.

Der Ulkus-Test wird wie folgt durchgeführt.

Es wurden männliche Wistar-Ratten (SPF) verwandt. Die Tiere liegen in einer Gewichtsspanne von 130±10 g. 16 Stunden vor Versuchsbeginn werden die Tiere vom Futter abgesetzt; sie erhalten Wasser ad libitum.

Pro Dosis werden 5 Tiere eingesetzt. Die Substanzen werden einmal oral, in Natriumchlorid gelöst oder als Kristallsuspension unter Zusatz von 85 mg% Myrj 53 appliziert.

3 Stunden nach Substanzapplikation injiziert man 1 ml einer 3%igen Lösung des Farbstoffs Diphenylreinblau intravenös und tötet das Tier. Der Magen wird reseziert und mikroskopisch auf Anzahl von Epithelläsionen und Ulcera, die durch Farbstoffanreicherungen hervortreten, untersucht.

Die nachfolgende Tabelle zeigt die in diesen Testen erhaltenen Ergebnisse der erfindungsgemäßen Verbindungen 6 und 7 im Vergleich zu den vorbekannten Substanzen 1 bis 5.

| Nr. | Verbindung | Adjuvans-Arthritis-Test in mg/kg Tier ($ED_{40}$) | Anzahl der Magenulcera bei gleicher Dosis |
|---|---|---|---|
| 1 | 2-(4-Isopropylphenyl)-propionsäure[1] (=Ibuprofen) | 100 | 6,8 |
| 2 | 2-(4-Cyclohexyl-phenyl)-propionsäure[2] | 40 | 7,6 |
| 3 | 5-Cyclohexyl-indan-1-carbonsäure[3] | 50 | 8,3 |
| 4 | 6-Chlor-5-cyclohexylindan-1-carbonsäure[3] | 4,0 | 7,8 |
| 5 | 2-(4-Cyclopropylmethyl-phenyl)-propionsäure[4] | 90 | 5,4 |
| 6 | 6-Chlor-5-cyclopentylmethyl-indan-1-carbonsäure | 30 | 2,2 |
| 7 | 6-Chlor-5-cyclopentyliden-methyl-indan-1-carbonsäure | 40 | 0,7 |

[1] US-Patent 3,385,886 [2] DOS 1 443 429, [3] J. Med. Chem., 1972, Vol. 15, 1297, [4] J. Med. Chem., 1973, Vol. 16, 487.

Die neuen Verbindungen eignen sich in Kombination mit den in der galenischen Pharmazie üblichen Trägermitteln zur Behandlung zum Beispiel von akuter und chronischer Polyarthritis, Neurodermitis, Asthma bronchiale, Heufieber u.a..

Die Herstellung der Arzneimittelspezialitäten erfolgt in üblicher Weise, indem man die Wirkstoffe mit geeigneten Zusätzen, Trägersubstanzen und Geschmackskorrigentien in die gewünschten Applikationsformen wie Tabletten, Dragees, Kapseln, Lösungen, Inhalationsmitteln usw. überführt.

Für die orale Anwendung eignen sich insbesondere Tabletten, Dragees und Kapseln, welche beispielsweise 1 bis 250 mg Wirkstoff und 50 mg bis 2 g pharmakologisch unwirksamen Trägers, wie zum Beispiel Laktose, Amylose, Talkum, Gelatine, Magnesiumstearat und ähnliches, sowie die üblichen Zusätze enthalten.

Die nachfolgenden Beispiele dienen zur Erläuterung des erfindungsgemäßen Verfahrens.

## Beispiel 1

a) 10 g 6-Chlorindan-1-carbonsäure werden in 100 ml absolutem Dichlormethan mit 12 g Aluminiumchlorid versetzt und auf —40°C gekühlt. In diese Mischung wird während 30 Minuten eine Lösung von 8,0 g 1,1-Dichlormethylmethyläther in 50 ml Dichlormethan eingetropft. Man rührt die Reaktionsmischung noch 30 Minuten lang bei —40°C, läßt sie erwärmen und gießt sie auf 100 g Eis unter Rühren. Dann trennt man die Dichlormethanphase ab, engt sie im Vakuum ein, kristallisiert den Rückstand aus Toluol um und erhält 8,9 g 6-Chlor-5-formyl-indan-1-carbonsäure vom Schmelzpunkt 162°C.

b) 5 g 6-Chlor-5-formylindan-1-carbonsäure werden mit 20 ml absolutem Äthanol und 1,5 ml konzentrierter Schwefelsäure versetzt und 4 Stunden lang unter Rückfluß erhitzt. Dann gießt man die Reaktionsmischung in 50 ml Wasser, extrahiert mit Chloroform, wäscht die Chloroformphase mit Wasser, trocknet sie über Natriumsulfat, engt sie im Vakuum ein, reinigt den Rückstand durch Destillation im Kugelrohr und erhält 4,2 g 6-Chlor-5-formylindan-1-carbonsäureäthylester vom Siedepunkt 150°C bei 0,054 mbar.

c) 304 mg 6-Chlor-5-formylindan-1-carbonsäureäthylester werden in 10 ml Äthanol gelöst und unter Rühren in eine Mischung von 21 mg Natriumborhydrid und 10 ml Äthanol eingetropft. Man rührt die Reaktionsmischung 4 Stunden lang bei 80°C und versetzt sie mit 50 ml 10%iger Schwefelsäure. Dann extrahiert man mit Chloroform, wäscht die Chloroformphase mit Wasser, trocknet sie über Natriumsulfat, engt sie im Vakuum ein und erhält 200 mg 6-Chlor-5-hydroxymethyl-indan-1-carbonsäureäthylester als Öl.

d) Eine Mischung aus 6,5 g Thionylchlorid, 5 ml Benzol und einem Tropfen Pyridin wird in eine Lösung von 1,2 g 6-Chlor-5-hydroxymethyl-1-carbonsäureäthylester eingetropft. Dann erhitzt man die Reaktionsmischung eine Stunde lang unter Rückfluß, läßt sie erkalten und gießt sie in Eiswasser. Die Benzolphase wird mit Wasser gewaschen, über Natriumsulfat getrocknet, im Vakuum eingeengt und man erhält 300 mg 6-Chlor-5-chlormethyl-indan-1-carbonsäureäthylester als Öl.

e) 2,2 g 6-Chlor-5-chlormethyl-indan-1-carbonsäureäthylester werden in 20 ml absolutem

Äthanol gelöst mit 1,38 g Kaliumsalz des Cyclopentan-2-on-1-carbonsäureäthylesters versetzt und 6 Stunden lang unter Rückfluß erhitzt. Dann setzt man der Reaktionsmischung 40 ml Wasser zu, extrahiert mit Äther, wäscht die Ätherphase mit Wasser, trocknet sie über Natriumsulfat und engt sie im Vakuum ein.

Der Rückstand wird 8 Stunden lang in 20 ml 10%iger wässriger Schwefelsäure unter Rückfluß erhitzt. Man läßt die Reaktionsmischung erkalten, versetzt sie bis zur alkalischen Reaktion mit verdünnter Natronlauge, extrahiert mit Äther säuert die wässrige Phase an und extrahiert sie nochmals mit Äther. Der Ätherextrakt der sauren Extraktion wird mit Wasser gewaschen, über Natriumsulfat getrocknet und man erhält die 6-Chlor-5-(2-oxocyclopentylmethyl)-indan-1-carbonsäure vom Schmelzpunkt 126°C (aus Petroläther).

Diese wird mit 15 ml Triglykol, einem Gramm Natriumhydroxid und 10 g Hydrazinhydrat versetzt, 2 Stunden lang auf 200°C erhitzt, mit Salzsäure angesäuert, und mit Chloroform extrahiert.

Die Chloroformphase wird mit Wasser gewaschen, über Natriumsulfat im Vakuum getrocknet und man erhält die 6-Chlor-5-cyclopentylmethyl-indan-1-carbonsäure als Öl.

NMR-Spektrum in Deuterochloroform:

Signale bei 1,5 ppm (mc, 9H); 2,6 ppm (mc, 6H); 3,9 ppm (T, J = 7 Hz, 1H); 7,0 ppm (s, 1H); 7,3 ppm (s, 1H).

## Beispiel 2

a) 7,45 g Cyclopentylbromid und 19,7 g Triphenylphosphin werden in einer Druckflasche unter Argon 6 Stunden in einem 160°C heißen Bad erhitzt. Nach Abkühlen wird das feste Reaktionsprodukt mehrmals mit Benzol ausgekocht und zum Schluß getrocknet. 15,7 g Cyclopentyltriphenylphosphoniumbromid werden erhalten.

b) 4,11 g Cyclopentyl-triphenylphosphoniumbromid werden in Tetrahydrofuran unter Argon suspendiert und bei 20°C mit 4,3 ml einer 3-molaren Lösung von Butyl-Lithium in n-Hexan versetzt. Nach 2 Stunden Rühren bei 20°C wird bei 5°C eine Lösung von 2,24 g 6-Chlor-5-formyl-indan-1-carbonsäure in 15 ml Tetrahydrofuran hinzugegeben. Nach 16 Stunden Rühren bei 20°C wird eingeengt, der Rückstand mit verdünnter Salzsäure versetzt und mit Äther extrahiert. Die Ätherphasen werden gewaschen eingeengt. Der Rückstand (2 g) wird über eine Kieselgelsäure chromatographiert (Elutionsmittel: Cyclohexan 325 Teile + Toluol 160 Teile + Essigester 190 Teile + Essigsäure 19 Teile). Man erhält nach Umkristallisation aus Benzin 1 g 6-Chlor-5-cyclopentylidenmethyl-indan-1-carbonsäure vom Schmelzpunkt 112°C.

c) 1,58 g 6-Chlor-5-cyclopentylidenmethyl-indan-1-carbonsäure werden bei 20°C und 760 Torr in 32 ml Äthanol nach Zugabe von 158 mg Platindioxid hydriert. Der Katalysator wird abfiltriert, das Filtrat eingeengt und der Rückstand aus Benzin umkristallisiert. 0,89 g 6-Chlor-5-cyclopentylmethyl-indan-1-carbonsäure von Schmelzpunkt 126°C werden erhalten.

## Beispiel 3

a) 0,50 g Cyclopentanon und 1,35 g 6-Chlor-5-formyl-indan-1-carbonsäure werden in einer Mischung aus 6,2 ml Essigsäure und 2 ml konzentrierter Schwefelsäure 1 Stunde bei 20°C gerührt. Danach wird auf Eiswasser gegossen und mit Äther extrahiert. Die Ätherphasen werden neutral gewaschen und eingeengt und der Rückstand über eine Kieselgelsäule chromatographiert (Cyclohexan 325 Teile + Toluol 160 Teile + Essigester 190 Teile + Essigsäure 19 Teile). 0,41 g 6-Chlor-5-(2-oxocyclopentyliden-methyl)-indan-1-carbonsäure vom Schmelzpunkt 170°C werden erhalten.

b) 2,5 g 6-Chlor-5-(2-oxocyclopentylidenmethyl)-indan-1-carbonsäure werden mit 1,3 g Hydrazinhydrat, 26 g Natriumhydroxyd und 40 ml Triglykol versetzt und zwei Stunden lang auf 200—220°C erhitzt. Nach Abkühlen wird mit Wasser versetzt, mit verdünnter Salzsäure angesäuert und mit Äther extrahiert. Die Ätherphasen werden mit Wasser gewaschen, eingeengt und der Rückstand aus Benzin umkristallisiert. 0,9 g 6-Chlor-5-cyclopentylmethyl-indan-1-carbonsäure vom Schmelzpunkt 126°C werden erhalten.

## Beispiel 4

a) Eine Lösung von 22,4 g 6-Chlor-indan-1-carbonsäureäthylester in 100 ml 1,2-Dichloräthan werden bei 0°C zu einer Mischung aus 28,4 g Cyclohexancarbonsäurechlorid, 26,6 g Aluminiumchlorid und 200 ml 1,2-Dichloräthan getropft. Nach 16 Stunden Rühren bei 20°C wird auf Eiswasser gegossen, und die organische Phase wird abgetrennt und eingeengt. Der Rückstand (49,2 g Öl) wird über Kieselgel chromatographiert (Elutionsmittel: Cyclohexan 95 Teile + Essigester 5 Teile). 3,7 g 6-Chlor-5-cyclohexylcarbonyl-indan-1-carbonsäureäthylester werden als Öl erhalten.

b) 3,3 g 6-Chlor-5-cyclohexylcarbonyl-indan-1-carbonsäureäthylester werden mit einer Mischung aus 1,06 g Natriumcarbonat, 2 ml Wasser und 6 ml Äthanol 2 Stunden gekocht. Ansäuern mit verdünnter Salzsäure bei 0°C und Absaugen ergibt 2,9 g 6-Chlor-5-cyclohexylcarbonyl-indan-1-carbonsäure vom Schmelzpunkt 67°C.

c) 1,3 g 6-Chlor-5-cyclohexylcarbonyl-indan-1-carbonsäure werden mit 0,65 g Hydrazinhydrat, 13 g Natriumhydroxyd und 20 ml Triäthylenglykol versetzt und 2 Stunden lang auf 200—220°C erhitzt. Abkühlen, Versetzen mit Wasser, Ansäuern mit verdünnter Salzsäure und Extraktion mit Äther

ergibt nach Einengen der Atherphase und Umkristallisation des Rückstandes aus Hexan 0,4 g 6-Chlor-5-cyclohexylmethyl-indan-1-carbonsäure vom Schmelzpunkt 131°C.

d) 0,36 g 6-Chlor-5-cyclohexylmethyl-indan-1-carbonsäure werden über 44 mg Palladium-Kohle (10%ig) in 10 ml Alkohol und 1 ml Wasser bei 20°C und 761 Torr hydriert. Nach Abfiltrieren des Katalysators und Einengen werden 0,27 g eines Öls erhalten. Präparative Schichtchromatographie auf Kieselgel (System: Cyclohexan-Essigester 1:1) ergibt 0,19 g 5-Cyclohexylmethyl-indan-1-carbonsäure vom Schmelzpunkt 54°C (aus Benzin).

**Patentansprüche**

1. Indan-1-carbonsäure-Derivate der allgemeinen Formel I

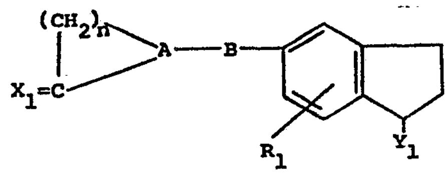

(I),

worin n die Ziffern 2 bis 5, 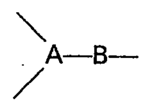

die Gruppierungen 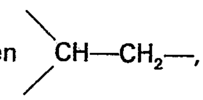, 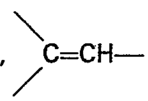

oder 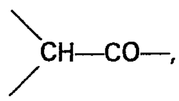,

$R_1$ ein Wasserstoffatom, ein Halogenatom, eine Trifluormethylgruppe, eine Nitrogruppe oder eine Aminogruppe,
$X_1$ zwei Wasserstoffatome oder eine Oxogruppe und
$Y_1$ eine Cyanogruppe, eine Hydroxamidocarbonylgruppe, eine Carbamoylgruppe, eine 5-Tetrazolylgruppe, eine Carboxylgruppe, deren Salze mit physiologisch verträgträglichen Basen, deren Ester von physiologisch unbedenklichen Alkoholen oder deren Amide von physiologisch unbedenklichen Aminen bedeuten.

2. 6-Chlor-5-(2-oxocyclopentyl-methyl)-indan-1-carbonsäure.
3. 6-Chlor-5-cyclopentylmethyl-indan-1-carbonsäure.
4. 6-Chlor-5-(2-oxocyclopentyliden-methyl)indan-1-carbonsäure.
5. 6-Chlor-5-cyclopentylmethyl-indan-1-carbonsäure.
6. 6-Chlor-5-cyclohexylcarbonyl-indan-1-carbonsäureäthylester.
7. 6-Chlor-5-cyclohexylmethyl-indan-1-carbonsäure.
8. 5-Cyclohexylmethyl-indan-1-carbonsäure.
9. 6-Chlor-5-cyclohexylcarbonyl-indan-1-carbonsäure.
10. Pharmazeutische Präparate, gekennzeichnet durch einen Gehalt an einer Verbindung gemäß Anspruch 1 bis 9 als Wirkstoff.
11. Verfahren zur Herstellung von Indan-1-carbonsäure-Derivaten der allgemeinen Formel Ia

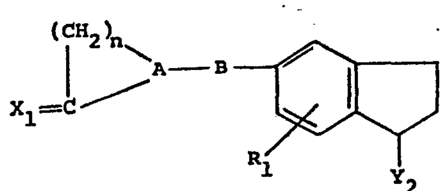

(Ia),

worin n, 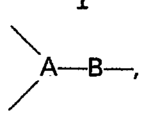,

$X_1$ und $R_1$ die gleiche Bedeutung wie in Anspruch I besitzen und $Y_2$ die gleiche Bedeutung wie $Y_1$ Besitzt, aber keine Cyanogruppe oder 5-Tetrazolylgruppe darstellt, dadurch gekennzeichnet, daß man in ansich bekannter Weise

a) ein Nitril der allgemeinen Formel II

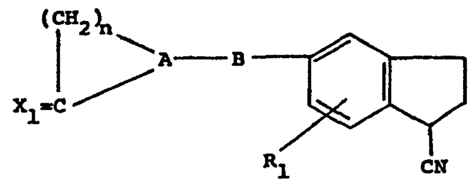 (II),

worin n, 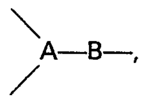

$X_1$ und $R_1$ die gleiche Bedeutung wie in Anspruch I besitzen hydrolysiert, oder

b) eine Verbindung der allgemeinen Formel III

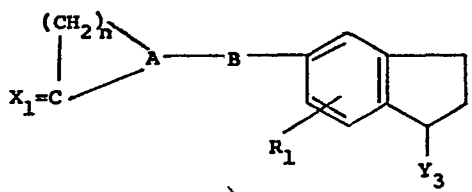 (III),

worin n, 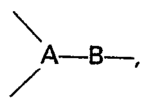

$X_1$ und $R_1$ die gleiche Bedeutung wie in Anspruch I besitzen und $Y_3$ eine Alkoxycarbonylgruppe, eine Dithianylidengruppe oder eine 4,4-Dimethyl-2-oxazolinylgruppe darstellt, hydrolysiert, oder

c) einen Aldehyd der allgemeinen Formel IV

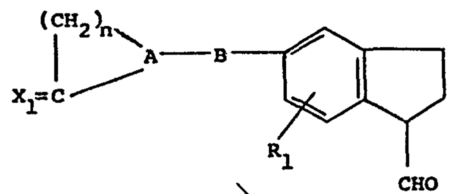 (IV),

worin n, 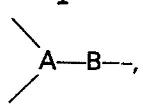

$X_1$ und $R_1$ die gleiche Bedeutung wie in Anspruch I besitzen oxidiert, oder

d) daß man die Oxogruppe einer Verbindung der allgemeinen Formel V

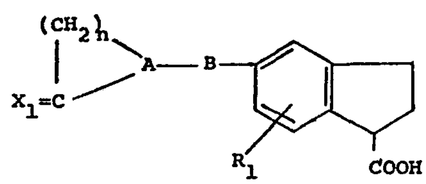 (V),

worin n, 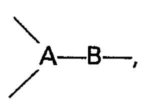

$X_1$ und $R_1$ die gleiche Bedeutung wie in Anspruch I besitzen, wobei mindestens eine der Gruppen A—B— oder C=$X_1$ eine Carbonylgruppe bedeutet, durch thermische Behandlung mit Hydrazin reduziert, oder

e) daß man ein Grignard-Reagenz der allgemeinen Formel VI

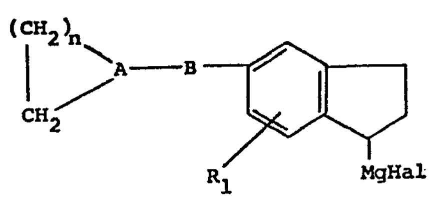 (VI),

worin n, 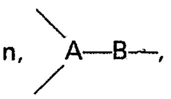

und $R_1$ die gleiche Bedeutung wie in Anspruch 1 besitzen und Hal ein Halogenatom darstellt, mit Kohlendioxid umsetzt, oder

f) daß man eine Verbindung der allgemeinen Formel VII

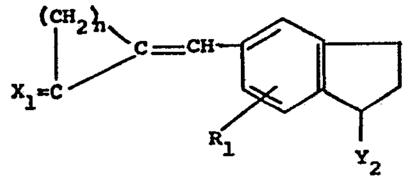

(VII),

worin
n, $X_1$, $Y_2$ und $R_1$ die gleiche Bedeutung wie in Anspruch I besitzen hydriert, oder

g) daß man eine Verbindung der allgemeinen Formel VIII

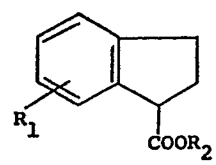

(VIII),

worin
$R_1$ die obengenannte Bedeutung besitzt und $R_2$ ein Wasserstoffatom oder einen Alkylrest mit 1 bis 6 Kohlenstoffatomen darstellt,
in Gegenwart von Friedel-Crafts Katalysatoren mit einem Cycloalkanoylchlorid der allgemeinen Formel IX

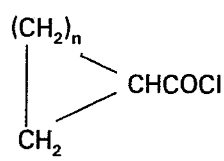

(IX),

worin n die obengenannte Bedeutung besitzt, kondensiert, oder

h) daß man eine Verbindung der allgemeinen Formel X

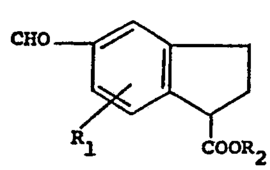

(X),

worin
$R_1$ die obengenannte Bedeutung besitzt und $R_2$ ein Wasserstoffatom der einen Alkylrest mit 1 bis 6 Kohlenstoffatomen darstellt,
mit einem Wittig Reagenz der allgemeinen Formel XI

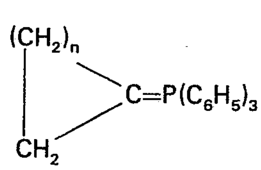

(XI),

worin n die obengenannte Bedeutung besitzt oder mit einer Carbonylverbindung der allgemeinen Formel XII

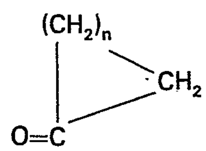

(XII),

worin n die obengenannte Bedeutung besitzt, kondensiert, oder

i) daß man eine Verbindung der allgemeinen Formel XIII

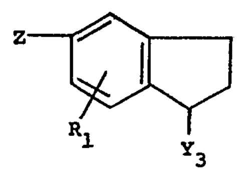

(XIII),

worin $R_1$ und $Y_3$ die obengenannte Bedeutung besitzen und Z eine Formylgruppe oder eine Cyanogruppe darstellt, mit einer metallorganischen Verbindung der allgemeinen Formel XIV

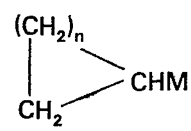

(XIV),

worin n die obengenannte Bedeutung besitzt und M ein Lithiumatom oder eine Magnesiumhalogenidgruppe darstellt umsetzt, oder

j) daß man eine Verbindung der allgemeinen Formel XV

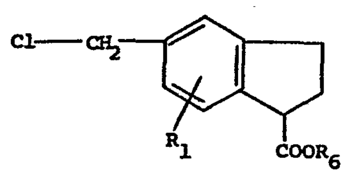

(XV),

worin $R_1$ und $R_6$ die obengenannte Bedeutung besitzen mit einem $\beta$-Ketoester der allgemeinen Formel XVI

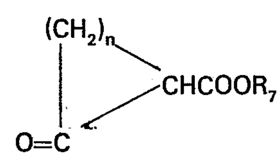

(XVI),

worin n die obengenannte Bedeutung besitzt und $R_7$ eine niedere Alkylgruppe bedeutet, kondensiert, die Estergruppen verseift und die entstandene $\beta$-Ketosäure decarboxyliert und gegebenenfalls Verbindungen der allgemeinen Formel Ia mit $R_1$ in der Bedeutung eines Wasserstoffatoms halogeniert oder nitriert und die erhaltenen Nitroverbindungen zu Aminoverbindungen reduziert, gegebenenfalls die erhaltenen Carbonsäuren oder reaktionsfähige Derivate derselben in ihre Salze, Ester, Amide oder Hydroxamsäuren überführt.

12. Verfahren zur Herstellung von Indan-1-carbonsäure-Derivaten der allgemeinen Formel Ib

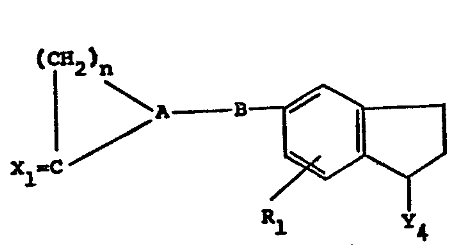

(Ib),

worin n, 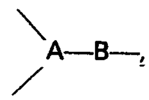

$X_1$ und $R_1$ die gleiche Bedeutung wie in Anspruch 1 besitzen und $Y_4$ eine Cyanogruppe, eine Carbamoylgruppe oder eine 5-Tetrazolylgruppe darstellt dadurch gekennzeichnet, daß man in ansich bekannter Weise

k) ein Keton der allgemeinen Formel XVII

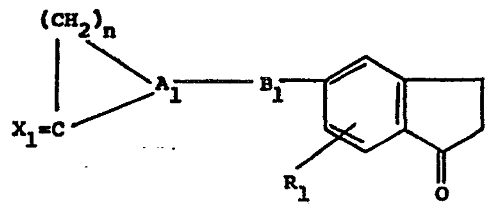

(XVII),

worin
n, $X_1$ und $R_1$ die gleiche Bedeutung wie in Anspruch 1 besitzen und 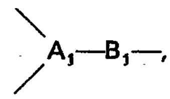

die Gruppierungen 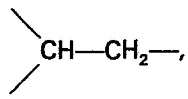 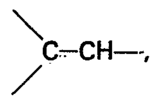

oder 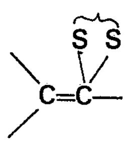

bedeutet mit einem Arylsulfonylmethylisocyanid umsetzt, oder

l) ein Halogenid der allgemeinen Formel XVIII

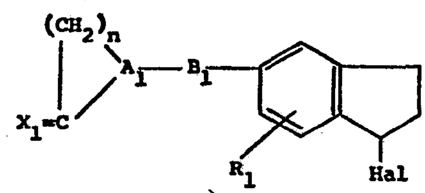

(XVIII),

worin n, 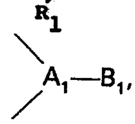

$X_1$ und $R_1$ die obengenannte Bedeutung besitzen und Hal ein Halogenatom darstellt, mit einem Alkalimetall cyanid umsetzt, oder

m) man eine Verbindung der allgemeinen Formel XIX

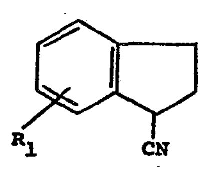

(XIX),

worin
$R_1$ die obengenannte Bedeutung besitzt in Gegenwart von Friedel-Crafts Katalysatoren mit einem Cycloalkanoylchlorid der allgemeinen Formel XX

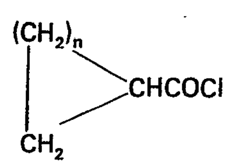

(XX),

worin n die obengenannte Bedeutung besitzt, kondensiert, gegebenenfalls vorhandene thioketalisierte Oxogruppen hydrolysiert und gegebenenfalls die erhaltenen Cyanide der allgemeinen Formel Ib zu den entsprechenden Amiden verseift oder sie in die entsprechenden Tetrazolylverbindungen überführt.

## Claims

1. Indane-1-carboxylic acid derivatives of the general formula I

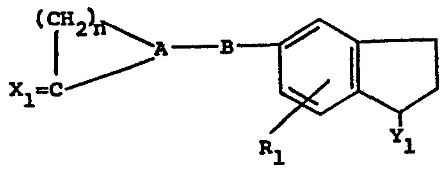

(I),

wherein n represents the numbers 2 to 5, >A—B— represents the groupings >CH—$CH_2$—, >C=CH— or >CH—CO—

$R_1$ represents a hydrogen atom, a halogen atom, a trifluoromethyl group, a nitro group or an amino group,

$X_1$ represents two hydrogen atoms or an oxo group, and

$Y_1$ represents a cyano group, a hydroxyamidocarbonyl group, a carbamoyl group, a 5-tetrazolyl group, a carboxyl group, the salts thereof with physiologically tolerable bases, the esters thereof with physiologically tolerable alcohols or the amides thereof with physiologically tolerable amines.

2. 6-chloro-5-(2-oxocyclopentylmethyl)-indane-1-carboxylic acid.
3. 6-chloro-5-cyclopentylmethylindane-1-carboxylic acid.
4. 6-chloro-5-(2-oxocyclopentylidenemethyl)-indane-1-carboxylic acid.
5. 6-chloro-5-cyclopentylmethylindane-1-carboxylic acid.
6. 6-chloro-5-cyclohexylcarbonylindane-1-carboxylic acid ethyl ester.
7. 6-chloro-5-cyclohexylmethylindane-1-carboxylic acid.
8. 5-cyclohexylmethylindane-1-carboxylic acid.
9. 6-chloro-5-cyclohexylcarbonylindane-1-carboxylic acid.
10. Pharmaceutical preparations, characterised by a content of a compound according to claims 1 to 9 as the active substance.
11. Process for the manufacture of indane-1-carboxylic acid derivatives of the general formula Ia

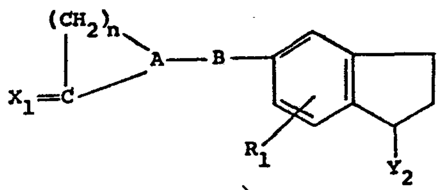

(Ia),

wherein n, >A—B—,

$X_1$ and $R_1$ have the same meanings as in claim 1 and $Y_2$ has the same meaning as $Y_1$ but is not a cyano group or a 5-tetrazolyl group, characterised in that, in a manner known *per se*,

a) a nitrile of the general formula II

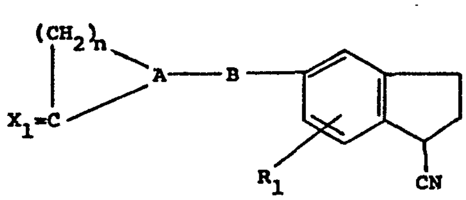

(II),

wherein n, A—B—,

$X_1$ and $R_1$ have the same meanings as in claim 1, is hydrolysed, or

b) a compound of the general formula III

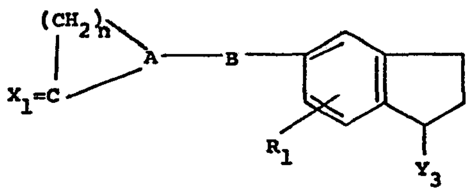

(III),

wherein n, A—B—,

$X_1$ and $R_1$ have the same meanings as in claim 1 and $Y_3$ represents an alkoxycarbonyl group, a dithianylidene group or a 4,4-dimethyl-2-oxazolinyl group, is hydrolysed, or

c) an aldehyde of the general formula IV

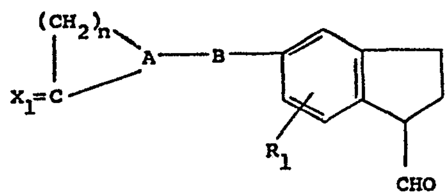

(IV),

wherein n, A—B—,

$X_1$ and $R_1$ have the same meanings as in claim 1, is oxidised, or

d) the oxo group of a compound of the general formula V

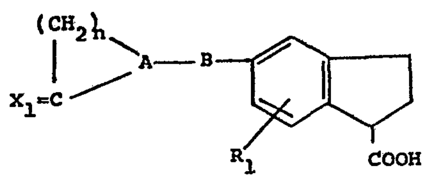

(V),

wherein n, A—B—,

$X_1$ and $R_1$ have the same meanings as in claim 1, at least one of the groups A—B— or $C{=}X_1$ representing a carbonyl group, is reduced by thermal treatment with hydrazine, or

e) a Grignard reagent of the general formula VI

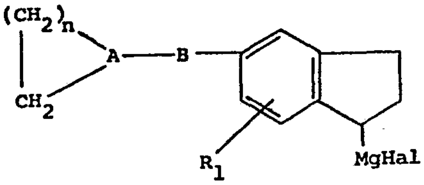

(VI),

wherein n, A—B—,

and $R_1$ have the same meanings as in claim 1 and Hal represents a halogen atom, is reacted with carbon dioxide, or

f) a compound of the general formula VII

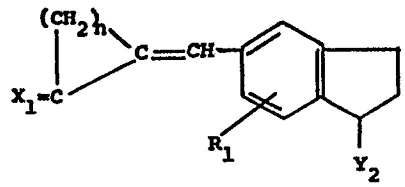

(VII),

wherein
n, $X_1$, $Y_2$ and $R_1$ have the same meanings as in claim 1, is hydrogenated, or
g) a compound of the general formula VIII

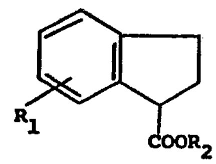

(VIII),

wherein
$R_1$ has the meaning given above and $R_2$ represents a hydrogen atom or an alkyl radical having from 1 to 6 carbon atoms, is condensed in the presence of Friedel-Crafts catalysts with a cycloalkanoyl chloride of the general formula IX

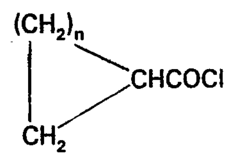

(IX),

wherein n has the meaning given above, or
h) a compound of the general formula X

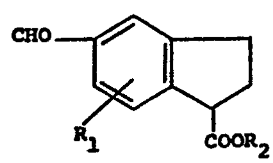

(X),

wherein
$R_1$ has the meaning given above and $R_2$ represents a hydrogen atom or an alkyl radical having from 1 to 6 carbon atoms, is condensed with a Wittig reagent of the general formula XI

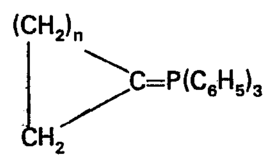

(XI),

wherein n has the meaning given above, or with a carbonyl compound of the general formula XII

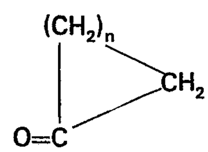

(XII),

wherein n has the meaning given above, or
i) a compound of the general formula XIII

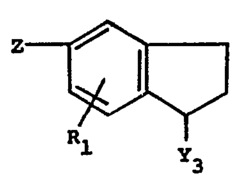

(XIII),

wherein

$R_1$ and $Y_3$ have the meanings given above and Z represents a formyl group or a cyano group, is reacted with an organometallic compound of the general formula XIV

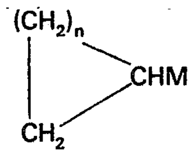

(XIV),

wherein n has the meaning given above and M represents a lithium atom or a magnesium halide group, or

j) a compound of the general formula XV

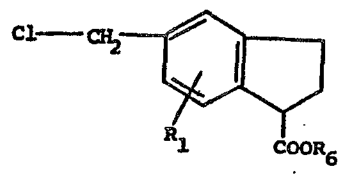

(XV),

wherein
$R_1$ and $R_6$ have the meanings given above, is condensed with a β-keto-ester of the general formula XVI

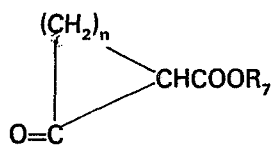

(XVI),

wherein n has the meaning given above and $R_7$ represents a lower alkyl group, the ester groups are hydrolysed and the resulting β-keto-acid is decarboxylated and, optionally, compounds of the general formula Ia where $R_1$ represents a hydrogen atom are halogenated or nitrated and the nitro compounds obtained are reduced to form amino compounds and, optionally, the carboxylic acids obtained or reactive derivatives thereof are converted into their salts, esters, amides or hydroxamic acids.

12. Process for the manufacture of indane-1-carboxylic acid derivatives of the general formula Ib

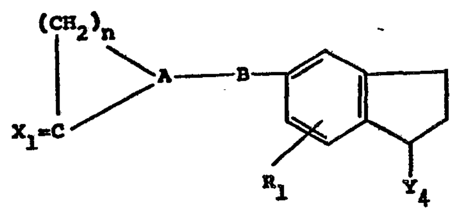

(Ib),

wherein n, >A—B—,

$X_1$ and $R_1$ have the same meanings as in claim 1 and $Y_4$ represents a cyano group, a carbamoyl group or a 5-tetrazolyl group, characterised in that, in a manner known *per se*,

k) a ketone of the general formula XVII

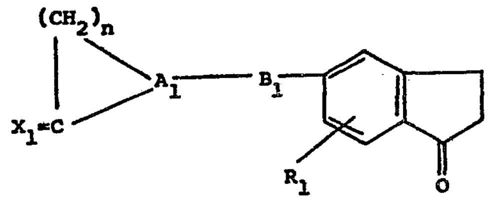

(XVII),

wherein n, $X_1$ and $R_1$ have the same meanings as in claim 1 and >$A_1$—$B_1$—

represents the groupings >CH—$CH_2$—, >C=CH—

or 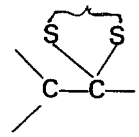

is reacted with an arylsulphonylmethyl isocyanide, or

l) a halide of the general formula XVIII

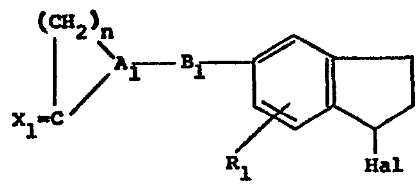 (XVIII),

wherein n, >$A_1B_1$—,

$X_1$ and $R_1$ have the meanings given above and Hal represents a halogen atom, is reacted with an alkali metal cyanide, or

m) a compound of the general formula XIX

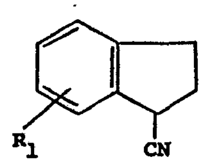 (XIX),

wherein

$R_1$ has the meaning given above, is condensed in the presence of Friedel-Crafts catalysts with a cycloalkanoyl chloride of the general formula XX

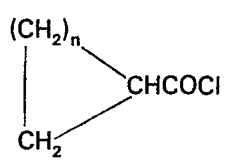 (XX),

wherein n has the meaning given above, thioketalised oxo groups optionally present are hydrolysed and, optionally, the cyanides obtained of the general formula Ib are hydrolysed to form the corresponding amides or are converted into the corresponding tetrazolyl compounds.

**Revendications**

1. Dérivés d'acides indane-carboxyliques-1 répondant à la formule générale I

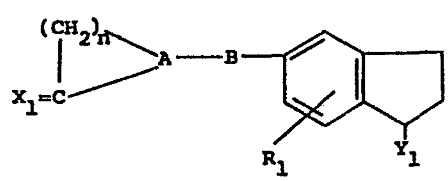 (I),

dans laquelle n désigne un nombre de 2 à 5, >A—B—

représente l'un des groupements >CH—$CH_2$—, >C=CH— et >CH—CO—,

$R_1$ représente un atome d'hydrogène ou d'halogène, un radical trifluoro-méthyle, un groupe nitro ou un groupe amino,

$X_1$ représente deux atomes d'hydrogène ou un groupe oxo et

$Y_1$ représente un groupe cyano, hydroxamido-carbonyle, carbamoyle ou tétrazolyle-5, un groupe carboxy ou l'un de ses sels avec des bases acceptables du point de vue physiologique, l'un de ses esters avec des alcools inoffensifs du point de vue physiologique ou l'un de ses amides dérivant d'amines sans inconvénients physiologiques.

2. Acide chloro-6 (oxo-2 cyclopentyl-méthyl)-5 indane-carboxylique-1.

3. Acide chloro-6 cyclopentylméthyl-5 indane-carboxylique-1.

4. Acide chloro-6 (oxo-2 cyclopentylidène-méthyl)-5 indane-carboxylique-1.

5. Acide chloro-6 cyclopentylidène-méthyl-5 indane-carboxylique-1.

6. Chloro-6 cyclohexylcarbonyl-5 indane-carboxylate-1 éthyle.

7. Acide chloro-6 cyclohexylméthyl-5 indane-carboxylique-1.

8. Acide cyclohexylméthyl-5 indane-carboxylique-1.

9. Acide chloro-5 cyclohexylcarbonyl-5 indane-carboxylique-1.

10. Compositions pharmaceutiques caractérisées en ce que'elles contiennent, comme substance active, un composé selon l'une quelconque des revendications 1 à 9.

11. Procédé de préparation de dérivés d'acides indane-carboxyliques-1 répondant à la formule générale Ia

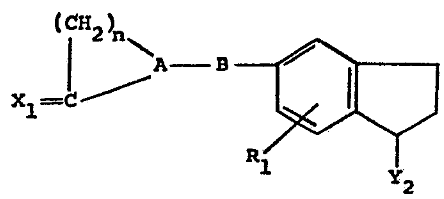

(Ia),

dans laquelle n, 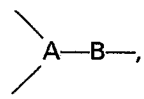

$X_1$ et $R_1$ ont les mêmes significations que dans la revendication 1 et $Y_2$ a la même signification que $Y_2$ mais ne peut représenter un groupe cyano ou tétrazolyle-5, procédé caractérisé en ce que, en opérant de manière connue:

a) on hydrolyse un nitrile répondant à la formule générale II

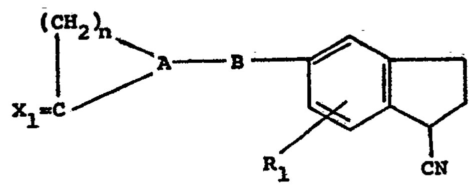

(II),

dans laquelle n, 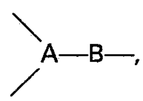

$X_1$ et $R_1$ ont les mêmes significations que dans la revendication 1, ou

b) on hydrolyse un composé répondant à la formule générale III

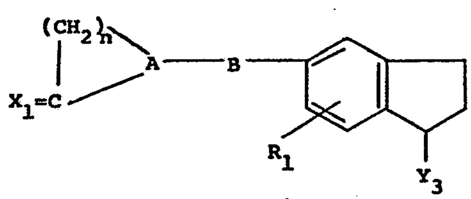

(III),

dans laquelle n, 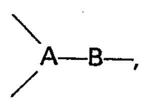

$X_1$ et $R_1$ ont les mêmes significations que dans la revendication 1 et $Y_3$ représente un radical alcoxy-carbonyle, dithiannylidène ou diméthyl-4,4 oxazolinyle-2, ou

c) on oxyde un aldéhyde répondant à la formule générale IV

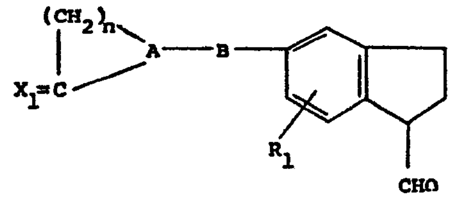

(IV),

dans laquelle n, >A—B—,

$X_1$ et $R_1$ ont les mêmes significations que dans la revendication 1, ou

d) on réduit le groupe oxo d'un composé répondant à la formule générale V

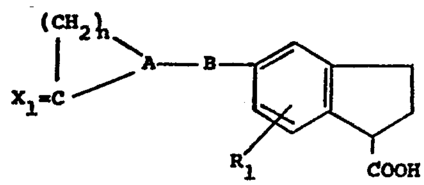

(V),

dans laquelle n, >A—B—,

$X_1$ et $R_1$ ont les mêmes significations que dans la revendication 1, l'un ou moins des groupes B

(de >A—B—)

et $C{=}X_1$ étant un groupe carbonyle, par traitement thermique avec l'hydrazine, ou

e) on fait réagir avec le dioxyde de carbone un réactif de Grignard répondant à la formule générale VI

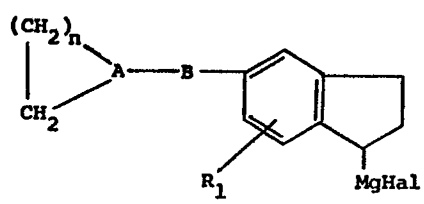

(VI),

dans laquelle n, >A—B—

et $R_1$ ont les mêmes significations que dans la revendication 1 et Hal représente un atome d'halogène, ou

f) on hydrogène un composé répondant à la formule générale VII

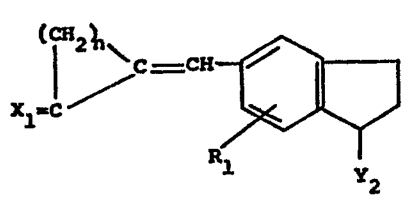

(VII),

dans laquelle n, $X_1$, $Y_2$ et $R_1$ ont les significations indiquées ci-dessus, ou

g) on condense un composé répondant à la formule générale VIII

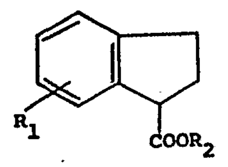

(VIII),

dans laquelle $R_1$ a la signification donnée à la revendication 1 et $R_2$ représente un atome d'hydrogène ou un radical alkyle contenant de 1 à 6 atomes de carbone, en présence de catalyseurs de Friedel-Crafts, avec un chlorure de cycloalcanoyle répondant à la formule générale IX

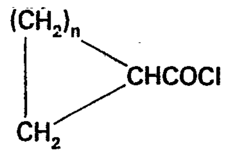

(IX),

dans laquelle n a la signification donnée à la revendication 1, ou

h) on condense un composé répondant à la formule générale X

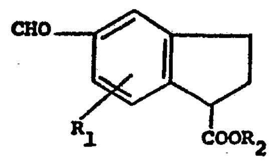

(X),

dans laquelle $R_1$ a la signification donnée à la revendication 1 et $R_2$ représente un atome d'hydrogène ou un radical alkyle contenant de 1 à 6 atomes de carbone, avec un réactif de Wittig répondant à la formule générale XI

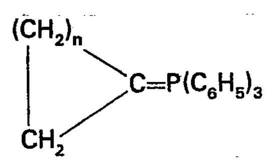

(XI),

dans laquelle n a la signification donnée ci-dessus, ou avec un composé carbonylique répondant à la formule générale XII

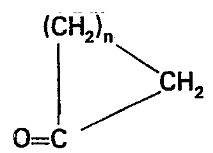

(XII),

dans laquelle n a la signification donnée ci-dessus, ou

i) on fait réagir un composé répondant à la formule générale XIII

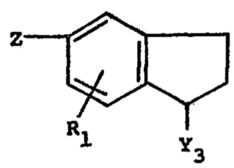

(XIII),

dans laquelle $R_1$ et $Y_3$ ont les significations indiquées ci-dessus et Z représente un radical formyle ou un groupe cyano, avec un composé organo-métallique répondant à la formule générale XIV

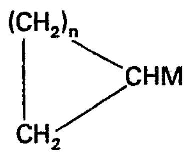

(XIV),

dans laquelle n a la signification indiquée ci-dessus et M représente un atome de lithium ou un groupe magnésium-halogénure, ou

j) on condense un composé répondant à la formule générale XV

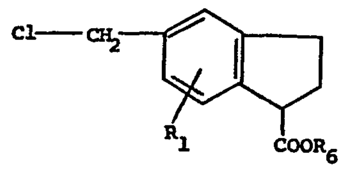

(XV),

dans laquelle $R_1$ et $R_6$ ont les significations précédemment données, avec un $\beta$-oxo-ester répondant à la formule générale XVI

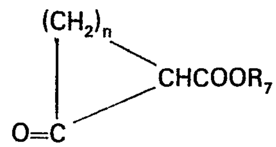

(XVI),

dans laquelle n a la signification précédemment donnée et $R_7$ représente un radical alkyle inférieur, on saponifie les groupes esters et on décarboxyle l'acide $\beta$-cétonique formé, et, le cas échéant, on halogène ou nitre des composés de formule générale Ia dans lesquels $R_1$ désigne un atome d'hydrogène et on réduit les composés nitrés obtenus pour les convertir en composés aminés, et éventuellement on transforme les acides carboxyliques obtenus ou leurs dérivés réactifs en leurs sels, esters, amides ou acides hydroxamiques.

12. Procédé de préparation de dérivés d'acides indane-carboxyliques-1 répondant à la formule générale Ib

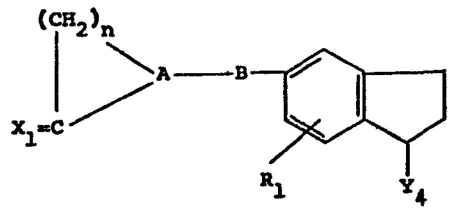

(Ib),

dans laquelle n, 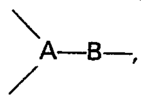,

$X_1$ et $R_1$ ont les mêmes significations que dans la revendication 1 et $Y_4$ représente un groupe cyano, un groupe carbamoyle ou un groupe tétrazolyle-5, procédé caractérisé en ce que, en opérant de manière connue:

k) on fait réagir une cétone répondant à la formule générale XVII

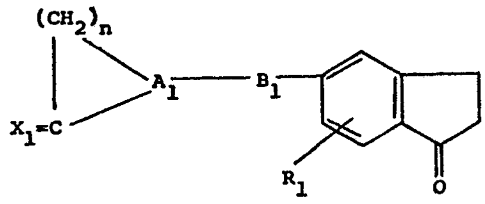

(XVII),

dans laquelle n, $X_1$ et $R_1$ ont les mêmes significations que dans la revendication 1 et 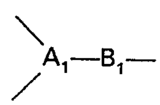

représente l'un des groupements suivants: 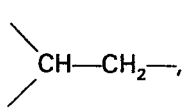, 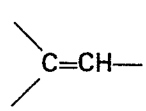

et 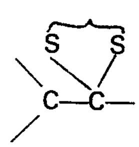

avec un isocyanure d'arylsulfonylméthyle, ou

l) on fait réagir un halogénure répondant à la formule générale XVIII

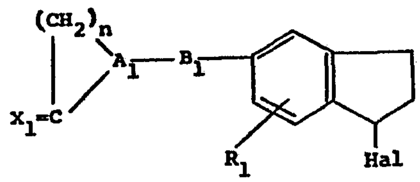

(XVIII)

dans laquelle n, 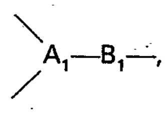 $X_1$ et $R_1$ ont les significations données ci-dessus et Hal représente un atome d'halogène, avec un cyanure de métal alcalin, ou

m) on condense un composé répondant à la formule générale XIX

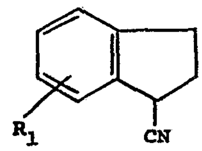

(XIX),

dans laquelle $R_1$ a la signification donnée ci-dessus, en présence de catalyseurs de Friedel-Crafts, avec un chlorure de cycloalcanoyle répondant à la formule générale XX

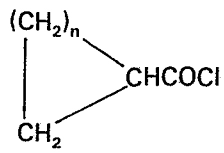

(XX),

dans laquelle n a la signification précédemment donnée, on hydrolyse d'éventuels groupes oxo thioacétalisés et éventuellement on saponifie les cyanures obtenus répondant à la formule générale Ib pour les convertir en les amides correspondants ou on les transforme en les composés tétrazolyliques correspondants.